Europäisches Patentamt

European Patent Office

Office européen des brevets

(11) Publication number: **0 291 479**
**A1**

(12) **EUROPEAN PATENT APPLICATION**

(21) Application number: 88850131.9

(22) Date of filing: 14.04.88

(51) Int. Cl.⁴: **G 01 N 33/571**
**G 01 N 33/543**

(30) Priority: 15.04.87 SE 8701571

(43) Date of publication of application:
**17.11.88 Bulletin 88/46**

(84) Designated Contracting States:
**AT BE CH DE ES FR GB GR IT LI LU NL SE**

(71) Applicant: **PHARMACIA AB**
**Rapsgatan 7**
**S-751 82 Uppsala (SE)**

(72) Inventor: **Nils, Thomas Hydén**
**Pilotgatan 21**
**S-122 51 Enskede (SE)**

**Thomas, Lars Olsson**
**Jättegrytsvägen 4**
**S-141 40 Huddinge (SE)**

The title of the invention has been amended (Guidelines for Examination in the EPO, A-III, 7.3).

(54) **Immunoassay method for the diagnosis of chlamydia infection.**

(57) Assay method for immunochemically detecting Chlamydia infections in mammals and birds, wherein bacteria of the genus Chlamydia potentially present in a sample are lysed in a lysing medium. The characteristic feature of this method consists in that an antigen released in the lysis step is adsorbed physically to a solid phase, and that a monoclonal anti-Chlamydia antibody preparation is contacted with the solid phase to which the antigen has been adsorbed. Conditions are arranged in such a manner that antibody-active components in the preparation are enabled to immunochemically bind to Chlamydia antigen that has been adsorbed to said solid phase. In a preferred embodiment the anti-Chlamydia antibody preparation is added to the lysing medium, thus avoiding a separation step.

The characteristic feature of the anti-Chlamydia antibody active components of the antibody preparation is that they consist of a plurality of antibody molecules and/or label groups bound covalently together.

Bundesdruckerei Berlin

**Description**

## Assay Method for the Diagnosis of Chlamydia infection

The invention is a novel assay for detecting infections with bacteria belonging to the genus Chlamydia, in particular Chlamydia trachomatis.

Bacteria of the genus Chlamydia are found as intracellular parasites in birds and mammals (including man). The genus comprises two species, via., C. trachomatis and C. psittaci. The former is the one occurring most commonly in humans; infections withC. trachomatis may cause a number of diseases in urogenital organs, eyes and the respiratory tract. As an example it may be mentioned that eye infections may be conducive to blindness, and infections in the urogenital organs of women may cause sterility. Nowadays infections with C. trachomatis are the most frequent ones among the sexually transmitted diseases. Often infected mothers will have transmitted the bacterium to their newborn child in whom the infection may then manifest itself in the form of eye inflammations or pneumonitis. In the urogenital organs the symptoms of Chlamydia infection are often vague and easily overlooked both by doctors and by patients. C. psittaci infections are less commonly occurring in humans. They are more often found in birds where C. psittaci is known in the first place for causing psittacosis - a disease which, as is also known, may when transmitted to humans give rise to serious infections in the respiratory tract. Up to now only one form of C. psittaci (serovar TWAR) has been discovered which is believed to be transmitted between humans (Grayston J.T. et al., N. Engl. J. Med. 315 (1981), 161-68).

By means of serological methods the genus Chlamydia has been subdivided into groups and strains (types, strains, serovars). C. trachomatis has 15 serovars. Thus bacteria of the genus Chlamydia have genus-, species-/group- and typespecific antigenic determinants present in their outer membrane exposed to their environment. These determinants (epitopes) may be positioned in different molecular units. In Chlamydia lipopolysaccharide (LPS) the existence of genus-specific determinants has been demonstrated (Stephens R. et al., J. Immunol. 128 (1982), 1083-89; Stephens R. et al., Thesis, Diss.Abstracts Int. 44 (1984), 3312 B; Caldwell H. et al., Inf. Immun. 44 (1984), 306-14, and EP-A-193,431). In the so-called MOMP protein (major outer membrane protein) type-and group-specific antigens have been demonstrated as well as genus- and species-specific antigens (Stephens R. et al. loc cit.; Caldwell MD et al., Inf. Immun. 31 (1981) and Batteiger B.E. et al., Inf. Immun. 52 (1986), 530-33). During recent years monoclonal antibodies have been produced which are directed against the said antigens, these antibodies having then been used in structural studies and diagnostic methods (see the articles mentioned above and also Zhang Y.-X. et al., J. Immunol. 138 (1987) 575-81).

There are several known methods for detecting infection with Chlamydia. Sampling is usually carried out with the aid of a stick carrying absorbent material, a so-called swab. Samples taken may be from eyes, nose, mouth, throat, urogenital organs etc. Such samples may originate from mammals of the Mammalia class, especially man, or from the Aves class. Since the bacteria have a largely intracellular life cycle it is imperative for the sample to contain both secretion (mucus) and cells from the sampling site. For this reason the composition of Chlamydia samples may vary considerably according to the particular sampling personnel, patients, sampling sites, time etc. in each case, and this in turn may give rise to difficulties for direct immunoanalyses of the original samples from the patients. A method commonly employed for determining whether or not bacteria are present in a sample relies on culturing; for in this manner the amount of the bacteria is increased so that their presence can then be detected more easily. Culturing of Chlamydia is a particularly laborious procedure inasmuch as their life cycle is largely intracellular and thus requires the use of suitable host cells. For the diagnosis of Chlamydia infections it has therefore been highly desirable to develop sufficiently sensitive assays applicable directly to the samples taken from the patients.

At present, a number of test systems are commercially available which are to serve for direct analysis of patients' samples. IDEIA® Chlamydia Test (Boots-Celltech Diagnostics Limited, Berkshire, Great Britain) and Chlamydiazyme® (Abbot Laboratories, North Chicago, Ill., USA) are two so-called solid phase methods. Both involve storing of the absorbed material with the sample in a transporting medium until analysis is to take place. In the first-mentioned test system the transporting medium contains agents causing the bacteria to lyse. When then the analysis is to be carried out the antigen that has been released is allowed to react with a genus-specific anti-Chlamydia monoclonal, this monoclonal having been adsorbed beforehand to the wall surface in a microtiter well. As a next step enzyme-labelled anti-Chlamydia monoclonal is added to the transporting medium in the well in order to detect Chlamydia antigen that has bound immunochemically to the wall of the well. This system uses a so-called enzyme cascade for enhancing its sensitivity, implying that the product formed by the antibody-bound enzyme has to participate in additional enzyme reactions to thus increase the strength of the signal. Chlamydiazyme® differs essentially from the IDEIA® Chlamydia Test, these two systems being methodologically different processes. The IDEIA® Chlamydia Test involves binding Chlamydia antigen immunochemically to the so-called solid phase (microtiter well). Due to the specificity in this adsorption the next step of the reaction sequence, i.e. addition of enzyme-labelled anti-Chlamydia monoclonal, can be carried out without preceding separation and washing. This is a procedure that is very often utilized in this type of tests (sandwich tests), see for example US-A-4,178,110. By contrast in the Chlamydiazyme® test the antigen present in the lysate is bound nonspecifically, by physical adsorption, to the solid phase. Processes of this type are usually called "coating" processes, the coating being always followed by a separation and washing procedure prior to the addition of antibody directed against the adsorbed antigen. The technique employed is such as in common practice in coating processes. Furthermore, in addition to these important methodological differences there are a number of minor features in which the two systems differ inter se. Thus in the case of Chlamydiazyme® it has to be noted also that (i) the solid consists of small beads, (ii) the anti-Chlamydia antibody preparation employed for detecting adsorption-bound antigen is polyclonal and unlabelled and (iii) anti-Chlamydia antibodies immunochemically bound to said beads are detected with the aid of an enzyme-labelled polyclonal anti-antibody preparation. The combination of two antibody preparations in Chlamydiazyme® results in an increased degree of sensitivity. This requires the use of an extra reagent and an extra incubation step.

Chlamydiazyme® has been described in US-A-4,497,899 which suggests also the use of vessel walls as an alternative to beads of plastics, and the use of monoclonal anti-Chlamydia trachomatis antibody as an alternative to the polyclonal anti-Chlamydia trachomatis antibody. US-A-4,497,899 does not mention that anti-Chlamydia antibody may be genus-specific and directed to Chlamydia-LPS.

Systems (sandwiches) similar to IDEIA® Chlamydia Test but focusing on one or more of the steps involved are disclosed in EP-A-183,383 (heating during the lysis), EP-A-167,395 and 174,106 (lysing solution) and EP-A-59,624 (a specific antigenic protein).

A study on the interference of some detergents on the binding of monoclonal antibodies to chlamydial antigens is disclosed in WO-A-83/03678 (p. 10-12).

In addition to the two aforesaid systems there are a number of further test systems available commercially. They rely on microscopy of sample smears which have been treated with fluorescence-labelled anti-Chlamydia antibodies (monoclonals).

The object of this invention is to provide a novel immunochemical testing methodology of detecting any potential infection with bacteria of the genus Chlamydia in samples of animal origin. Test procedures within the concept of this invention may be carried out more quickly and with lesser reagent requirements while having potentially a degree of sensitivity at least equal to that of prior art procedures.

The invention is an immunochemical assay for detecting infection with bacteria of the genus Chlamydia in birds and mammals, primarily humans. In this process bacteria of the genus Chlamydia potentially present in a sample are lysed in a lysing medium. The process is characterized in that Chlamydia antigen present in the lysing medium is then adsorbed physically to a suitable solid phase, e.g. a surface of plastics like for instance the wall of a microtiter well, dispersible plastics particles, larger beads etc., in order to then be detected immunochemically on the solid phase. Chlamydia antigen detected on the solid phase is taken as an indication that the sample contains the antigen and consequently that the animal from which the sample has been taken is infected with bacteria of the genus Chlamydia. According to the invention, the detection of the adsorbed antigen is carried out in that after said adsorption a monoclonal anti-Chlamydia antibody preparation is added so that anti-Chlamydia antibody-active components in said antibody preparation can react immunochemically with a Chlamydia antigen which has been adsorbed to the solid phase. Antibody-active component that has bound to the solid phase immunochemically is taken as an indication that the sample has contained Chlamydia antigen. The antibody preparation may be added to the lysing medium without separation of said medium from the solid phase.

The anti-Chlamydia antibody-active components employed are in the form of covalent antibody aggregates (multi-conjugates, multi-antibodies) in which a plurality ($>1$) of antibody molecules and/or label groups are bound together, for example via glutaraldehyde or disulfide linkages. The aggregates should contain more than 3, e.g. more than 6 or 10 antibody molecules bound together covalently (either intact or fragmented molecules). The number of marker groups in the aggregate should amount to at least one per antibody molecule. Preferably the molecular weight of the aggregate is more than $10^6$ daltons.

The term "solid phase" refers to a phase that is insoluble in the reaction media employed and is capable of physically adsorbing the antigen contemplated. Usually the solid base is hydrophobic. No capturing antibody is employed, nor any other component possessing biospecific affinity for the Chlamydia antigen.

It is really surprising that it is thus possible to eliminate the separation step normally following after an adsorption step, while at the same time maintaining or improving the degree of sensitivity. During the adsorption step conditions should be such as to give an optimum exposure of hydrophobic structures in the antigen, whereas in the step bestowing specificity on the test system (incubation step with antibody) the said exposure should be kept at a minimum.

Lysing medium

As mentioned above samples for clinical diagnoses of Chlamydia infections are taken with a so called swab which is then kept sealed in a suitable transporting medium. After transportation and just before analysis is to be carried out the bacteria in the sample are lysed in a suitable medium. The process of lysing bacteria of the Chlamydia genus is prior art technique, see for example US-A-4,497,899 and the IDEIA® Chlamydia Test. From such knowledge as has been acquired up to now it may be inferred that it will be advantageous in the case of the present invention, as in the process described in US-A-4,497,899, to employ detergents such as salts or deoxycholate or other bile acids etc. Lysing is performed for the purpose of increasing sensitivity.

At present the detergent considered to be the most advantageous for the process of this invention is chenodeoxycholate. Detergent concentration in the lysing medium may vary depending on the particular Chlamydia antigen to be detected. For chenodeoxycholate in combination with monoclonal anti-MOMP antibodies one should choose a detergent concentration of 0.2-1 % (w/v). When monoclonals directed against LPS are employed the detergent concentration is a more critical factor, in the case of chenodeoxycholate the concentration should be within the range of about 2.0-2.6 mM, for example 2.3 mM. Since MOMP is held together by disulfide bridges it is advantageous to use reducing agents like dithiothreitol, if MOMP is the target protein to be detected.

Lysis is best carried out at 25-100 °C, preferably with rapid boiling on a water bath followed by cooling. The pH will vary with the detergent employed; in the case of chenodeoxycholate the pH should be in the range of 7-12. The lysing medium is as a rule buffered with phosphate or carbonate, but of course other buffering systems may be employed provided they do not interfere with the test. As a general rule the lysing conditions chosen should be such that they will give maximum sensitivity and precision in an optimal way. An important point to be remembered is that the pH and buffer capacity should be chosen such as to permit an easy adjustment to the pH value which is necessary for the immune reaction.

### Anti-Chlamydia antibody preparation

The term "anti-Chlamydia antibody preparation" means that the antibody preparation is directed against genus-, species-, group- or type-specific antigen to be found principally in the genus Chlamydia. The said antigen thus must not occur in other bacteria frequently encountered in connection with the type of diagnostics for which a given antibody preparation is to be used (see for instance Science 220 (1983), 1279-81).

The preparation are monoclonal and may consist of a single monoclonal, preferably a genus- or species-specific monoclonal (e.g. for C. trachomatis), or of a mixture of a finite number of monoclonals (e.g. less than 10, like 2, 3, 4, or 5 each specific for either a species, a group or a type of bacteria belonging to the genus Chlamydia. Here the intended use of the test is the factor that will decide which combination is to be chosen. In the case of human samples taken in urogenital organs only C. trachomatis is to be expected; for this reason it is possible to use a genus- or trachomatis-specific monoclonal, although also combination of group- and type-specific monoclonals may be acceptable. If the intention is to diagnosticate C. psittaci in cage birds (e.g. parrots, for instance budgerigars) a monoclonal specific for C. psittaci will be the appropriate choice even though good results might also be obtained with a genus-specific monoclonal (as far as is known C. trachomatis does not occur in parrots).

Producing monoclonals directed against genus-, species-, group- or type-specific Chlamydia antigens is prior art technique, and this has been done in several laboratories all over the world. See for example the articles cited above. The monoclonals are also available commercially.

The antibody-active components of the preparations may possess a covalently bound analytically detectable group, as e.g. an enzymatically active, radioactive, chemiluminescent, fluorescent, biotin etc. group. Labelled components (if present in the preparation) can be detected on the solid phase after having been bound thereto immunochemically. In cases where the components employed are unlabelled they can be detected in the usual manner with the aid of so-called anti-antibodies which in turn may be provided with analytically detectable groups. The antibody-active components of the preparation may also be used in the form of antibody-active fragments of suitable monoclonals, e.g. Fab, Fab' or F(ab')$_2$ fragments.

According to what has been ascertained up to the present date, the best results with the invention are obtained with monoclonals directed against the genus-specific epitope of Chlamydia-LPS.

The immune reactions are carried out at a pH and temperature such as are normally employed in immunochemical assays; that is, the pH should be 5-8.6 and the temperature should range from 0 to 40 °C, preferably 4 to 40 °C. Reaction media should be buffered with buffer systems having a high buffering capacity with the aforesaid pH range, which means they will have to correspond to an acid-base pair having a pKa within said range. To prevent non-specific adsorption of anti-Chlamydia antibody-active components the reaction medium should contain an effective amount of an agent that will block such adsorption. An example are inert proteins. A person skilled in the art will find no difficulty in obtaining the optimum concentration by titration; in the case of proteins such as e.g. BSA or some equivalent protein this concentration is below about 2 % (w/v) but above 0.2 %, preferably above 1 % (w/v).

The invention is defined in the attached claims which form an integral part of this specification and will now be illustrated by means of patent examples.

### Reagent solutions

Solution for lysing and adsorbing ("coating") antigen onto microtiter wells: chenodeoxycholate 2.3 mM (= 1 mg/ml), NaCl 50 mM, $PO_4^{3-}$ 0.1 M, pH 7.1.

Solution for adsorbing non-lysed material to microtiter wells (so-called $CO_3$ coating): $CO_3^{2-}$ 50 mM, pH 9.6.

Wash solution: $PO_4^{3-}$ 15 mM, NaCl 0.15 M, Tween® 20 0.05 % and pH 7.0.

Substrate solution (ALP): p-nitrophenyl phosphate 3.3 mg/ml, diethanolamine 1.04 M, $MgCl_2$ 0.1 mg/ml, and pH 9.8.

Transporting solution: $PO_4^{3-}$ 15 mM, NaCl 200 mM and pH 7.1.

Solution for monoclonal-ALP conjugate (ALP = alkaline phosphatase): Bovine serum albumin (BSA) 50 mg/ml, Tris 100 mM, Tween® 20 0.05 %, $MgCl_2$ 1 mM, $ZnCl_2$ 0.1 mM and pH 8.0.

Preparing the ALP conjugate: This was done in that monoclonal antibody and ALP (alkaline phosphatase, Sigma, St.Louis, Miss., USA) were each reacted separately with a molar excess (>5) of SPDP (N-succinimidyl

4

3-(2-pyridyldithio)-propionate, Pharmacia AB, Sweden) for introducing 2-pyridyldisulfide groups. The pyridyldisulfide groups in ALP-SPDP were then reduced to free thiol groups, and the resultant thiolated ALP was subjected to a thiol-disulfide exchange reaction with the monoclonal SPDP derivative. The synthesis was carried out in accordance with the instructions for SPDP except that all reaction steps were performed in 20 mM $PO_4^{3-}$, 0.15 M NaCl at pH 7.2 (see also US-A-4,232,119). By gel chromatography the conjugate recovered was found to have a molecular weight exceeding $10^6$ daltons indicating that at least 3 antibody molecules are linked covalently together. The conjugate was thus a so-called multiconjugate (US-A-4,232,119).

By varying the molar ratio between SPDP, enzyme and antibody the molecular weight of the conjugate can be varied.

Antimouse-ALP conjugate was from Dupont New England Nuclear, Boston, Mass., USA. The substrate solution for the enzyme was $PO_4^{3-}$ 50 mM, BSA 5 mg/ml, NaCl 150 mM, Tween® 20 0.05 % and pH 7.1.

Europium chelate-monoclonal conjugate: Europium complex with isothiocyanate phenyl EDTA was coupled to monoclonal anti-Chlamydia antibody in accordance with the prior art method of Lövgren, T. et al. (Alternative Immuno-assays, Ed. Collins, W.P., John Wiley & Sons Ltd (1985), 203-17).

Horseradish-monoclonal conjugate: Peroxidase from horseradish (HRP, Sigma Chemical Co., St. Louis, Miss., USA) was coupled after activation with periodate in accordance with the prior art method of Boorsma and Strefkeerk (J. Immunol. 30 (1979), 245-55).

## Producing monoclonal antibody

### Antigen
Partially purified and intact Chlamydia trachomatis serovar $L_2$ ("elementary bodies", $L_2$EB), strain 434-Bu (Abo University), and egg-cultured material ($L_2$) (Statens Bakteriologiska Laboratorium, Sweden).

### Immunization scheme:
FCA = Freuds Complete Adjuvant. FIA = Freuds Incomplete Adjuvant.

3-4 immunization at intervals of 3 weeks. 1st: FCA subcut. + foot pad. 2nd: FIA subcut. + ip. 3rd: FIA subcut. + ip. 50-70 µg of protein per injection.

### Mice:
Strain Balb/c (Statens Bakteriologiska Laboratorium, Sweden) aged $\geq$ 5 weeks.

The mice were bled and their sera were titrated onto $L_2$EB coating (microtiter wells coated with C. trachomatis $L_2$EB 3 days after the second and 2 days after the third immunization. ALP-antimouse antibody was used for detecting antibodies that had been adsorbed to the wells. The best serum after the third immunization decided which mouse was to be selected.

### Fusing method
A selected mouse was killed 3 days after the third immunization, and its spleen was taken for being fused with the myeloma cell line sp2/0 (Flow Laboratories, Irvine, Cal., USA) as according to de St. Groth et al. (Journal of Immunological Methods 35 (1980), 1-21) with PEG 4000 (Merck, Darmstadt, West Germany).

The cell line was recloned twice and was cultured with intervening freezing and thawing for a total period of 10 months without any decrease in titer or change in the IEF pattern (IEF = isoelectric focusing).

### Characterizations of the selected monoclonal:
(1) The subclass was determined by means of catcher-ELISA methodology using isotype-specific antisera as the capturing antibody, $IgG_1$, k.

(2) The titer was determined by means of ELISA technique on a $CO_3$ coating of $L_2$EB. Result: $10^{-3}$ for supernatant from culture and $10^{-6}$ for ascites fluid.

(3) Antibody production of the individual clones was determined by means of quantitative catcher-ELISA of supernatants from stationary phase cultures and of ascitic fluid that had been purified by means of chromatography (protein A, ion exchange chromatography). Result: 5 µg/ml (supernatant from culture) and 1.5 mg/ml (ascites fluid).

(4) Mab IEF pattern on agarose gel gave a pI of pH 7.0-7.5.

(5) Reactivity assay by means of indirect immunofluorescence microscopy, carried out with all serotypes of Chlamydia trachomatis. Result: genus reactivity.

(6) Western blot performed with the lysate of cell-cultured bacteria showed the following:

    a) Reactivity against LPS.

    b) A genus typical LPS determinant.

    c) The antibody is not inhibited by ketodeoxyoctanoic acid in ELISA; this applies also to doses > 20 µg/ml.

    d) Purified LPS fractions from Salmonella minnesota Re mutant totally deficient in 0-antigen do not show any reaction with the monoclonal. Similarly LPS from Salmonella typhimurium does not give any reaction.

## EXAMPLES

Examples of test protocols of procedures according to the invention:

Clinical samples are taken with a so-called sampling stick, and during the time until they are analyzed they are stored in a transporting tube together with 0.1 ml of transporting solution. After transportation of the tube to the laboratory for analysis 0.5 ml of lysing medium is added, the tube is shaken vigorously and then the stick is removed. Thereafter the tube is heated on a boiling water bath; after cooling, it is ready for analysis. From each sample 100 µl are allowed for 30 minutes to become adsorbed to the plastics wall surfaces of microtiter wells. The temperature is 37 °C. To the wells containing the lysing solution there are then added 50 µl of ALP-monoclonal conjugate, and the mixture is incubated for 45 minutes at 37 °C. After his incubation the wells are washed 4 times with wash solution, and 100 µl of substrate solution are added. Reading of $OD_{405nm}$ takes place after 60 minutes. Values higher than those of the blank (0.05 Abs, see above) are interpreted as being Chlamydia-positive.

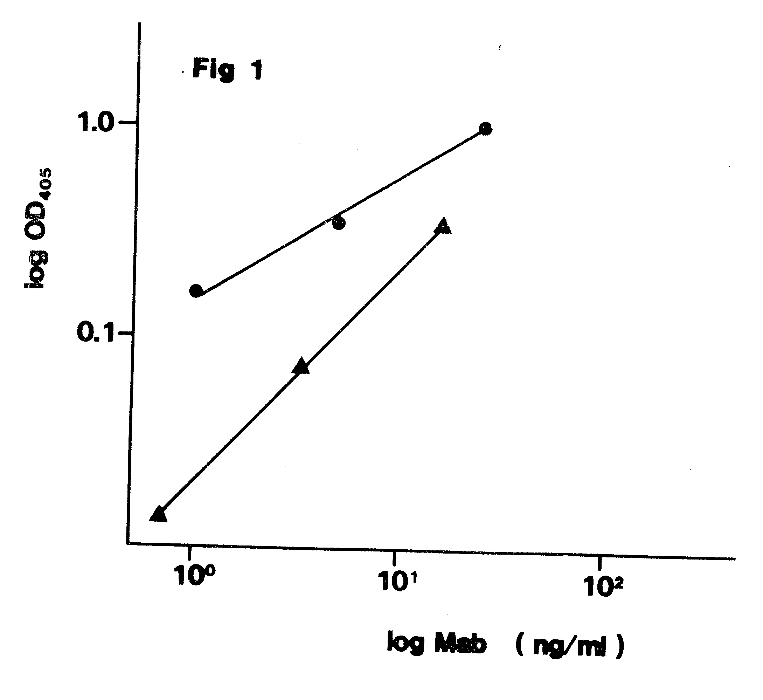

A. The above test protocol was carried out with a constant content of Chlamydia antigen (protein 30 ng/ml) and was compared with a protocol where washing (3 times with PBS-Tween) was carried out in the stage between the addition of sample and addition of conjugate (Fig. 1). The result shows that the analysis can be rendered more sensitive when no washing step is included.

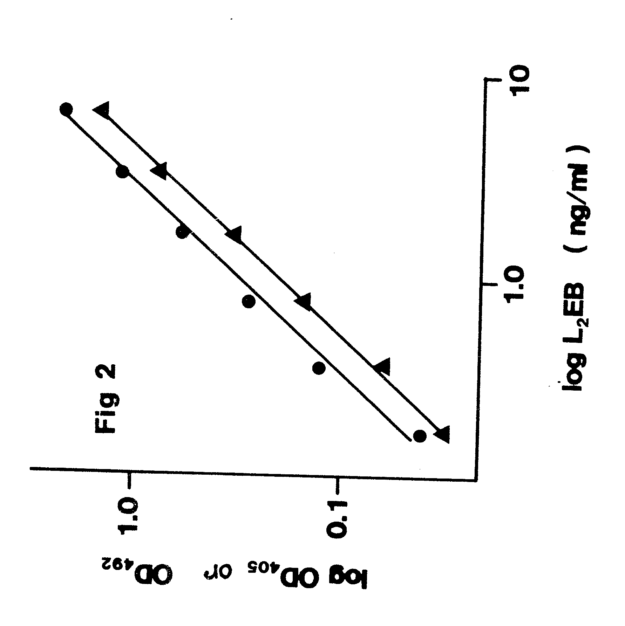

B. The above test protocol was carried out in tests where isolated Chlamydia antigen was added in varying amounts (according to protein determination). The same additions were analyzed in Chlamydiazyme®; the comparison is represented in Fig. 2. The results show that the invention can give an analytical sensitivity that compares very well with Chlamydiazyme®.

C. The above test protocol was then applied to a clinical situation; the results obtained were compared with conventional culturing and with Chlamydiazyme®. The methods showed very good agreement inter se.

| Results: | Chlamydiazyme | | | | | Invention | | |
|---|---|---|---|---|---|---|---|---|
| | | + | − | | | | + | − |
| c | | | | | c | | | |
| u | | | | | u | | | |
| l | + | 34 | 3 | | l | + | 34 | 4 |
| t | | | | | t | | | |
| u | | | | | u | | | |
| r | − | 7 | 146 | | r | − | 7 | 145 |
| e | | | | | e | | | |

The method of the invention was deemed to have very great practical advantages over Chlamydiazyme® because it gives the same sensitivity with fewer reagents and fewer reaction steps.

D. The experiments set forth below will illustrate the reactivity of the antibody with C. trachomatis serovar $L_2$ elementary bodies ($L_2$EB) and LPS from C. trachomatis as compared to LPS from Salmonella rough, E. coli and Acinetobacter (the latter may occur in urogenital samples and its LPS is similar to Chlamydia LPS). Test protocol as above. Concentrations refer to the transporting solution/lysing medium mixture.

After development for 60 minutes with p-nitrophenyl phosphate the $OD_{405nm}$ was measured. Results:

| Antigen | Conc.* | $OD_{405nm}$ |
|---------|--------|--------------|
| Chl-L$_2$EB | 1 ng/ml | 0.950 |
| Chl-LPS | 1 ng/ml | 1.850 |
| Salmonella rough LPS | 1 /ug/ml | 0.050 |
| Acinetobacter LPS | 1 /ug/ml | 0.050 |
| E. coli LPS | 1 /ug/ml | 0.040 |
| Uncoated | | 0.050 |

* protein in the case of L$_2$EB, dry weight for the rest.

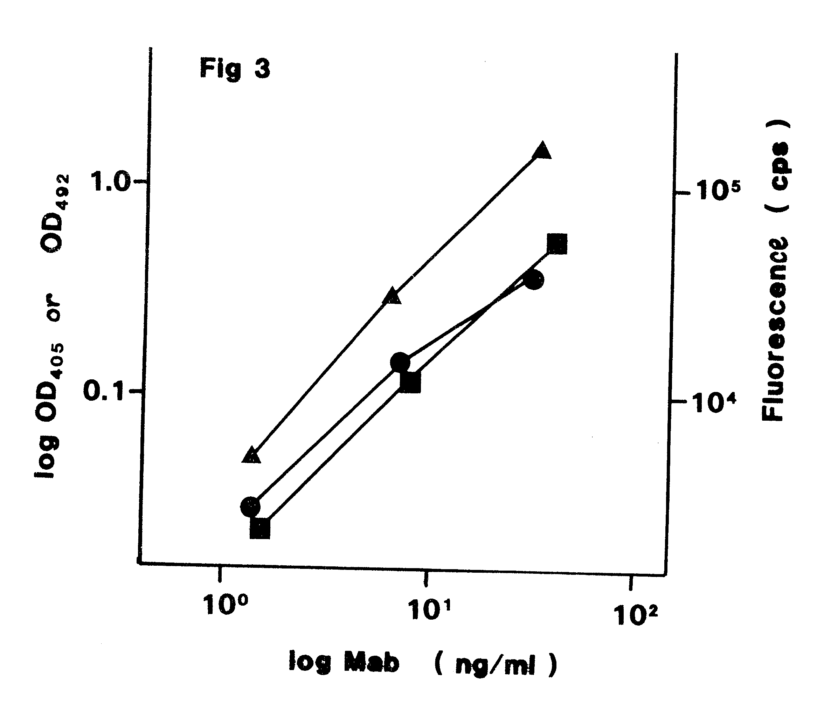

E. Different markers (HRP, ALP or europium chelate) were bound to the monoclonal antibody, and different amounts of the conjugates were tested with $CO_3$-adsorbed antigen (Fig. 3). The experiment showed that all the conjugates were active.

F. For a direct evaluation of the significance of (a) the monoclonal antibody and (b) elimination of the first washing step in Chlamydiazyme® the latter test was performed without a washing step. Results indicated that this would lower the sensitivity of the test, that is, in a clinical situation the number of false negatives would rise.

To sum up, the experiments show that anti-Chlamydia antibodies which are monoclonal and are directed against e.g. genus-specific epitopes in Chlamydia LPS give very great and unexpected advantages.

Figures

Fig. 1 illustrates the above experiment A. Circles indicate values measured in the case of the invention, while triangles indicate values obtained in the experiments in which separating and washing steps have been included.

Fig. 2 illustrates the above experiment B. Circles represent the invention ($OD_{405}$), and triangles represent Chlamydiazyme® ($OD_{492}$).

Fig. 3 illustrates experiment E. Triangles represent runs with peroxidase ($OD_{492}$), circles represent runs with ALP ($OD_{405}$), and squares represent runs with Eu chelate (cps).

## Claims

1. Assay method for immunochemically detecting Chlamydia infections in mammals and birds, wherein bacteria of the genus Chlamydia potentially present in a sample are lysed in a lysing medium, characterized in that

(i) Chlamydia antigen which after said lysis is present in the lysing medium is adsorbed physically to a solid phase which is insoluble in the lysing medium,

(ii) in connection with the lysing step, preferably after said adsorption has taken place, a monoclonal anti-Chlamydia antibody preparation is allowed to react immunochemically with Chlamydia antigen that has been adsorbed to said solid phase, and thereafter

(iii) an antibody-active component originating from said antibody preparation and bound immunochemically to said solid phase is detected in a manner known per se and taken as an indication that the mammal or bird from which the sample has been obtained is afflicted with a Chlamydia infection;

and in that the anti-Chlamydia antibody active components of said antibody preparation is in the form of aggregates in which a plurality of antibody molecules and/or label groups are bound together covalently.

2. Method according to claim 1, characterized in that said antibody preparation is directed against a Chlamydia LPS epitope which is unique to Chlamydia.

3. Method according to claim 2, characterized in that said epitope is genus-specific.

4. Method according to any of claims 1-3, characterized in that the anti-Chlamydia antibody-active components of said anti-Chlamydia antibody preparation contain at least three antibody units which are bound together inter se covalently.

5. Method according to any of claims 1-4, characterized in that the antibody-active components of said anti-Chlamydia antibody preparation are provided with an analytically detectable group.

6. Method according to any of Claims 1-5, characterized in that the sample is of human origin, preferably urogenital.

7. Method according to any of Claims 1-6, characterized in that said antibody preparation is added to the lysing medium without a preceding separation of said medium from said solid phase.

Fig 1

log OD$_{405}$

log Mab ( ng/ml )

Fig 2

log OD$_{405}$ or OD$_{492}$

1.0

0.1

1.0

10

log L$_2$EB   ( ng/ml )

Fig 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 497 899 (A.S. ARMSTRONG et al.) * whole document * --- | 1-6 | G 01 N 33/571 G 01 N 33/543 |
| Y | CLINICAL CHEMISTRY, vol. 22, no. 8, 1976, pages 1243-1255; G.B. WISDOM: " Enzyme-Immunoassay " * see in particular page 1249, left col., lines 16-17 and 27-28 * --- | 1,4,5 | |
| Y | EP-A2-175 560 (E.I. DU PONT DE NEMOURS AND COMPANY) * page 3, lines 11-20 * --- | 1,4,5 | |
| A | METHODS IN ENZYMOLOGY, vol. 73, 1981, pages 147-166; M.J. O'SULLIVAN et al.: "Methods for the preparation of Enzyme-Antibody Conjugates for Use in Enzyme Immunoassay". * see in particular pages 148-149 * --- | 1,4,5 | |
| A | EP-A1-183 383 (IQ (BIO) LTD) * example 1 and col. 2; lines 35-42 * --- | 1-6 | |

**TECHNICAL FIELDS SEARCHED (Int. Cl.4)**

G 01 N

The present search report has been drawn up for all claims

| Place of search | Date of completion of the search | Examiner |
|---|---|---|
| STOCKHOLM | 30-06-1988 | GUSTAFSSON C.O. |